# EUROPEAN PATENT SPECIFICATION

(11) **EP 3 185 376 B1**
(45) Date of publication and mention of the grant of the patent: **14.09.2022**
(21) Application number: 15842285.7
(22) Date of filing: 24.06.2015
(51) Int. Cl.: H01T 23/00, A61L 9/22, H01T 19/04, F24F 8/30, F24F 8/192

(54) **DISCHARGE UNIT**
ENTLADUNGSEINHEIT
UNITÉ DE DÉCHARGE

(30) Priority: 19.09.2014 JP 2014191366; 03.06.2015 JP 2015113165
(43) Date of publication of application: 28.06.2017
(73) Proprietor: Daikin Industries, Ltd., Osaka-shi, Osaka 530-8323 (JP)
(72) Inventor: SUZUMURA, Kei, Osaka-shi, Osaka 530-8323 (JP); KAWABATA, Kazuyoshi, Osaka-shi, Osaka 530-8323 (JP); URABE, Daisuke, Osaka-shi, Osaka 530-8323 (JP)
(74) Representative: Goddar, Heinz J.
(86) International application number: PCT/JP2015/003175
(87) International publication number: WO 2016/042691

(56) References cited:
- EP-A1- 1 659 665
- JP-A- H0 330 847
- JP-A- H0 330 847
- JP-A- 2005 216 539
- JP-A- 2009 076 416
- JP-A- 2009 224 280
- JP-A- 2010 086 790
- JP-A- 2013 045 531
- JP-A- 2014 029 821
- JP-U- H 079 943
- JP-U- H 079 943

## Description

### TECHNICAL FIELD

The present invention relates to a discharge unit.

### BACKGROUND ART

Discharge units which cause a discharge between a discharge electrode and a counter electrode have been known. For example, a discharge unit disclosed in Patent Document 1 is mounted in an air conditioner. The discharge unit includes a discharge electrode having a discharge needle (i.e., a discharge section), a counter electrode facing the tip of the discharge needle, and a voltage supply section which applies a potential difference between these electrodes. When a voltage is supplied to the discharge electrode from the voltage supply section, a streamer discharge is generated from the tip of the discharge needle toward the counter electrode. Due to this streamer discharge, active species (e.g., electrons, ions, radicals and ozone) are generated in the air. These active species decompose harmful or odor components in the air.
Patent document 2 and Patent document 3 each disclose a discharge unit comprising a discharge electrode, wherein the discharge electrode is configured as a metal material and includes a plate-like electrode support plate and a plurality of discharge sections arranged along, and supported at, a side edge portion of the electrode support plate so as to be opposed to the counter electrode, a counter electrode, a voltage supply section configured to give a potential difference between the discharge electrode and the counter electrode, and a resin-made insulating member which supports the discharge electrode and the counter electrode and includes a support which supports a middle range of the electrode support plate between two outermost discharge sections of the plurality of discharge sections.
Patent document 4 relates to an ozone generator for generating ozone to be used for deodorizing, sterilizing, etc. applications. One end portion of the ozone generator in a longitudinal direction thereof is fixed by caulking.
Patent document 5 relates to a discharge unit and air cleaning apparatus using the same. The discharge unit includes an insulating substrate, a semi conductive portion provided on the surface of the insulating substrate on the front end side of the discharge electrode, and a semi-A counter electrode formed on a conductive portion provided on the insulating substrate, a discharge electrode arranged to penetrate the circular hole portion of the insulating substrate, a support member and a conductive portion hole supply connection portion and a power supply portion for applying a voltage to the power supply connection portion and the discharge electrode. The support member is composed of a fixed lid, a base for fixing the counter electrode so as to sandwich it from both sides and a base plate for fixing the discharge electrode.
Patent document 6 introduces the possibility of a stabilizing conductive support component.

### CITATION LIST

### PATENT DOCUMENT

Patent Document 1: Japanese Unexamined Patent Publication No. 2014-119186
Patent document 2: JP H03 30847 A
Patent document 3: JP 2005 216539 A
Patent document 4: JP H07 9943 U
Patent document 5: JP 2014 029821 A
Patent document 6: EP 1 659 665 A1

### SUMMARY OF THE INVENTION

### TECHNICAL PROBLEM

The inventors of the present application have reached a structure of the above-described discharge unit in which a metal discharge electrode and a counter electrode are supported by a resin insulating member. This structure allows the discharge electrode and the counter electrode to have a uniform relative distance between each other, and allows insulation between the discharge electrode and the counter electrode. The inventors of the present application have also reached a structure in which a discharge section is supported on a side edge portion of a plate-like electrode support plate and in which both ends of the electrode support plate are supported by an insulating member.

However, a problem is that these support structures cannot keep a suitable distance between the discharge section and the counter electrode. This is because the resin insulating member has a thermal expansion coefficient greater than the metal electrode support plate. For this reason, if an ambient temperature of the discharge unit changes during the fabrication or use of the discharge unit, the electrode support plate and the insulating member may expand or contract to have different lengths. Specifically, if an ambient temperature of the discharge unit drops abruptly in winter, for example, the insulating member contracts more than the electrode support plate. As a result, the electrode support plate may warp in the thickness direction, which leads to a problem in which a suitable distance between the electrode support plate and the counter electrode, and hence between the discharge section and the counter electrode, cannot be kept.

In view of the foregoing background, it is therefore an object of the present invention to reduce the risk of being unable to keep a suitable distance between a discharge section and a counter electrode due to ambient temperature changes.

### SOLUTION TO THE PROBLEM

A first aspect of the present invention is directed to a discharge unit according to claim 1.

According to the first aspect of the invention, the metal discharge electrode (70) and the counter electrode (60) are supported by the resin-made insulating member (41). As a result, relative positions between the discharge electrode (70) and the counter electrode (60) are determined, and the discharge electrode (70) and the counter electrode (60) are insulated from each other.

The discharge electrode (70) is configured such that a plurality of discharge sections (73, 74) are supported at a side edge portion of the electrode support plate (71). A discharge from the tips of the discharge sections (73, 74) to the counter electrode (60) occurs when the voltage supply section (30) gives a potential difference between the discharge electrode (70) and the counter electrode (60).

The support (47) of the insulating member (41) supports the middle region (71a) of the electrode support plate (71). The middle region (71a) is part of the electrode support plate (71) between two outermost discharge sections (73a, 74a) of the plurality of discharge sections (73, 74). In other words, the support (47) of the insulating member (41) does not support both end portions of the electrode support plate (71), but supports the middle region of the electrode support plate (71). If the support (47) of the insulating member (41) supports both the end portions of the electrode support plate (71), the electrode support plate (71) may warp easily due to ambient temperature changes as mentioned above. On the other hand, the support (47) of the insulating member (41) of the present invention supports the middle region (71a) of the electrode support plate (71). Thus, even if thermal shrinkage of the insulating member (41) occurs, the middle region (71a) of the electrode support plate (71) does not warp severely. Accordingly, even if the ambient temperature changes, the distance between the discharge sections (73, 74) and the counter electrode (60) does not change much.

A second aspect of the invention is an embodiment of the first aspect. In the second aspect, the electrode support plate (71) has an elongated shape extending in a direction of arrangement of the plurality of discharge sections (73, 74).

According to the second aspect, the electrode support plate (71) extends in a direction of arrangement of the plurality of the discharge sections (73, 74). As such, a large number of discharge sections (73, 74) may be provided at the electrode support plate (71), which increases the amount of active species generated by a discharge.

The electrode support plate (71) having an elongated shape may warp easily due to ambient temperature changes. However, the electrode support plate (71) is less likely to warp compared to the configuration supporting both ends of the electrode support plate (71), since the support (47) of the insulating member (41) supports the middle region (71a) of the electrode support plate (71). Accordingly, even if the ambient temperature changes, the distance between the discharge sections (73, 74) and the counter electrode (60) does not change much.

The above-listed Patent Document 1 discloses a discharge electrode in which an edge of a support plate is folded and crimped to hold and support a discharge section in the support plate (see FIG. 3 of Patent Document 1). This configuration requires a complicated process for forming the support structure of the discharge section. In addition, a problem may occur that the discharge section is bent or broken and a suitable distance between the discharge section and the counter electrode cannot be kept.

To address this problem, in the present invention, the middle region (71a) of the electrode support plate (71) of the discharge electrode (70) is supported by being sandwiched between the insulating member (41) and the fixing member (80). The support structure is thus easily formed. Further, almost no load acts on the discharge sections (73, 74), and the discharge sections (73, 74) are thus prevented from being bent or broken. As a result, the accuracy in determining a distance between the discharge sections (73, 74) and the counter electrode (60) is improved.

According to the present invention, the fixing member (80) is configured as a conductive member (80) having a flanged portion (86). The flanged portion (86) is arranged opposite the counter electrode (60) with the discharge sections (73, 74) interposed therebetween, has the same electric potential as the discharge sections (73, 74), and has a shape along the discharge sections (73, 74).

The flanged portion (86) is opposed to the counter electrode (60) with the discharge sections (73, 74) interposed therebetween. The flanged portion (86) has the same electric potential as the discharge sections (73, 74), and has a shape along the discharge sections (73, 74). The flanged portion (86) of the conductive member (80) may stabilize the discharges directed to the counter electrode (60) from the discharge sections (73, 74).

The middle region (71a) of the electrode support plate (71) is supported by being sandwiched between the conductive member (80) and the insulating member (41). That is, the conductive member (80) serves as a member which stabilizes discharges and also as a fixing member which stably supports the discharge electrode (70).

A third aspect of the invention is an embodiment of the first aspect. In the third aspect, the middle region (71a) of the electrode support plate (71) is provided with an opening (72). The insulating member (41) has a fitted portion (49) fitted in the opening (72).

According to the third aspect, the fitted portion (49) of the support (47) is fitted in the opening (72) of the middle region (71a) of the electrode support plate (71). This structure provides reliable positioning between the electrode support plate (71) and the insulating member (41), and hence between the discharge electrode (70) and the counter electrode (60).

A fourth aspect of the invention is an embodiment of the third aspect. In the fourth aspect, the opening (72) has an elongated shape extending in a direction of arrangement of the plurality of discharge sections (73, 74) along the side edge portion of the electrode support plate. The fitted portion (49) has an elongated shape so as to be fitted in the opening (72).

According to the fourth aspect, the opening (72) of the middle region (71a) has an elongated shape extending in the direction of arrangement of the discharge sections (73, 74). The fitted portion (49) of the insulating member (41) has an elongated shape corresponding to this shape of the opening (72). Such a structure in which a portion of the middle region (71a) supported by the fitted portion (49) has an increased dimension in the longitudinal direction of the electrode support plate (71), may effectively prevent warpage of the middle region (71a) of the electrode support plate (71).

A fifth aspect of the invention is an embodiment of the third or fourth aspect. In the fifth aspect, the support (47) includes a support body (48) and a projection (49) as the fitted portion which protrudes upward from an upper end of the support body (48). A ring-shaped, planar mounting surface (50) surrounding the projection (49) is formed on the upper end of the support body (48), on which the electrode support plate (71) is mounted.

The insulating member (41) of the fifth aspect has the support body (48) and the projection (49) which protrudes from the upper end of the support body (48). The projection (49) serves as the fitted portion. The projection (49) of the insulating member (41) is fitted in the opening (72) of the electrode support plate (71), thereby having the middle region (71a) of the electrode support plate (71) supported on the insulating member (41).

The insulating member (41) is provided with a ring-shaped mounting surface (50) surrounding the projection (49) on the upper end of the support body (48). The electrode support plate (71) is placed on the mounting surface (50). The mounting surface (50) is a planar surface which contributes to maintaining the flatness of the electrode support plate (71). Hence, the mounting surface (50) may keep an optimum distance between the plurality of discharge sections (73, 74) and the counter electrode (60).

A sixth aspect of the invention is an embodiment of any one of the third to fifth aspects. The conductive member (80) includes a cylindrical wall portion (81) in which the fitted portion (49) is fitted and which supports the flanged portion (86).

According to the sixth aspect, the fitted portion (49) of the insulating member (41) is fitted in the cylindrical wall portion (81) of the conductive member (80). Thus, relative positions among the insulating member (41), the discharge electrode (70), and the conductive member (80) are determined, which in turn determines relative positions between the discharge sections (73, 74) and the counter electrode (60), and between the discharge sections (73, 74) and the flanged portion (86). The electrode support plate (71) is supported by being sandwiched between the insulating member (41) and the cylindrical wall portion (81).

A seventh aspect of the invention is an embodiment of the sixth aspect. In the seventh aspect, the fitted portion (49) is fixed to the cylindrical wall portion (81) by ultrasonic wave welding.

According to the seventh aspect, the fitted portion (49) fitted in the cylindrical wall portion (81) is melted by an ultrasonic wave. The melted resin material adheres to an inner circumferential surface and other surfaces of the cylindrical wall portion (81), and solidifies. As a result, the conductive member (80) and the insulating member (41) are firmly fixed to each other, and the electrode support plate (71) is sandwiched between the cylindrical wall portion (81) and the insulating member (41) with reliability.

### ADVANTAGES OF THE INVENTION

According to the present invention, the insulating member (41) supports the middle region (71a) of the electrode support plate (71), which may reduce the risk of warpage of the electrode support plate (71). This structure may maintain an optimum distance between the discharge sections (73, 74) and the counter electrode (60), and achieve continuous generation of desired stable discharges.

According to the second aspect of the invention, a large number of discharge sections (73, 74) may be provided at a side edge portion of the electrode support plate (71), which increases the amount of active species generated by a discharge. As a result, the air purifying properties of the discharge unit may be improved.

According to the invention, the electrode support plate (71) may be stably supported by the insulating member (41), and the flatness of the electrode support plate (71) may be kept. As a result, the optimum distance between the discharge sections (73, 74) and the counter electrode (60) may be maintained.

According to the invention, the middle region (71a) of the electrode support plate (71) is supported by being sandwiched between the insulating member (41) and the fixing member (80), which may simplify the support structure for the discharge sections (73, 74) and reduce the cost and time for forming the support structure. Further, this structure may prevent the discharge sections (73, 74) from bending and generate stable discharges.

According to the invention, the provision of the flanged portion (86) so as to be opposed to the counter electrode (60) with respect to the discharge sections (73, 74) may stabilize the discharge directed to the counter electrode (60) from the discharge sections (73, 74). The conductive member (80) having the flanged portion (86) serves also as a fixing member which sandwiches the electrode support plate (71) between itself and the insulating member (41). This structure may reduce the number of components.

According to the third aspect of the invention, the discharge sections (73, 74) and the counter electrode (60) may be positioned by fitting the fitted portion (49) in the opening (72) of the electrode support plate (71). In addition, the electrode support plate (71) may be supported on the insulating member (41) by a simple structure.

According to the fourth aspect of the invention, the warpage of the middle region (71a) may be more effectively prevented by fitting the elongated fitted portion (49) into the elongated opening (72) formed in the middle region (71a).

According to the fifth aspect of the invention, the insulating member (41) is provided with the flat mounting surface (50) formed on the support body (48) and surrounding the projection (49), and the electrode support plate (71) is placed on the mounting surface (50). The flatness of the electrode support plate (71) may thus be maintained, which may maintain the optimum distance between the discharge sections (73, 74) and the counter electrode (60).

According to the sixth aspect of the invention, the relative positions among the flanged portion (86), the discharge sections (73, 74), and the counter electrode (60) may be determined by inserting the fitted portion (49) in the cylindrical wall portion (81) of the conductive member (80). As a result, more stable discharges may be generated from the discharge sections (73, 74) to the counter electrode (60). Further, the cylindrical wall portion (81) may be used as a spacer for keeping a distance between the flanged portion (86) and the discharge sections (73, 74).

According to the seventh aspect of the invention, the conductive member (80) and the insulating member (41) may be firmly fixed to each other by welding, using an ultrasonic wave, the fitted portion (49) fitted in the cylindrical wall portion (81). The discharge electrode (70) may thus be supported between the conductive member (80) and the insulating member (41) with reliability.

### BRIEF DESCRIPTION OF THE DRAWINGS

FIG. 1 is a schematic diagram illustrating an air conditioner of an embodiment.
FIG. 2 is a perspective view of a casing of a discharge unit, when viewed from the front side.
FIG. 3 is a perspective view of the casing of the discharge unit, when viewed from the rear side.
FIG. 4 is a perspective view of an internal structure of the discharge unit.
FIG. 5 is an assembly diagram of a discharge process section and its peripheral devices.
FIG. 6 is a plan view of the discharge process section and its peripheral devices for illustrating a state in which the discharge electrode and a stabilizer are removed from the discharge process section.
FIG. 7 is a plan view of the discharge process section and its peripheral devices for illustrating a state in which the stabilizer is removed from the discharge process section.
FIG. 8 is a plan view of the discharge process section and its peripheral devices.
FIG. 9 is a cross-sectional view of FIG. 8 taken along the plane IX-IX.
FIG. 10 is a perspective view of the stabilizer.
FIG. 11 is a diagram when viewed along the arrow XI of FIG. 8.
FIG. 12 is a plan view of a discharge electrode.
FIG. 13 is a cross-sectional view of FIG. 8 taken along the plane XIII-XIII.
FIG. 14 is a plan view of a discharge electrode according to a variation of the embodiment.
FIG. 15 is a perspective view of a stabilizer according to a variation of the embodiment.
FIG. 16 is an enlarged plan view of a discharge section according to a first example of another embodiment.
FIG. 17 is an enlarged plan view of a discharge section according to a second example of another embodiment.
FIG. 18 is an enlarged plan view of a discharge section according to a third example of another embodiment.

### DESCRIPTION OF EMBODIMENTS

Embodiments of the present invention will be described in detail below, with reference to the drawings. The following embodiments are merely exemplary ones in nature, and are not intended to limit the scope, applications, or use of the invention.

A discharge unit (20) of the present invention is mounted in an air conditioner (10). The air conditioner (10) adjusts the temperature of air in an indoor space (S).

### <Configuration for Air Conditioner>

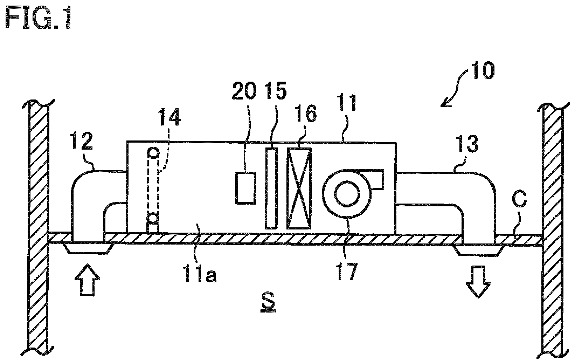

As illustrated in FIG. 1, the air conditioner (10) is installed on the back side of a ceiling (C). The air conditioner (10) has an air conditioner casing (11) having a horizontally-oriented box-like shape. A room-air duct (12) is connected to one of the longitudinal side surfaces of the air-conditioner casing (11). An air-supply duct (13) is connected to the other longitudinal side surface of the air-conditioner casing (11). An air passage (11a) is formed in the interior of the air-conditioner casing (11). The room-air duct (12) has an intake end communicating with the indoor space (S) and an outlet end communicating with the air passage (11a). The air-supply duct (13) has an intake end communicating with the air passage (11a) and an outlet end communicating with the indoor space (S).

The air passage (11a) is provided with a prefilter (14), a discharge unit (20), a catalyst filter (15), a heat exchanger (16), and a fan (17) sequentially arranged from the upstream to downstream side of the air flow (i.e., from the room-air duct (12) to the air-supply duct (13)). The prefilter (14) catches relatively-large dust in the air. In the discharge unit (20), active species are generated by a discharge, and these active species decompose harmful or odor components in the air.

The catalyst filter (15) is comprised, for example, of a base with a honeycomb structure which carries a catalyst on its surface. A manganese-based catalyst or a noble metal-based catalyst is used as the catalyst. The catalyst filter (15) further activates the active species generated by the discharge to promote decomposition of the harmful or odor components in the air. The catalyst filter (15) carries an adsorbent (e.g., activated carbon) which adsorbs the harmful or odor components in the air.

The heat exchanger (16) heats and cools the air flowing through the air passage (11a). Specifically, the heat exchanger (16) is connected to a refrigerant circuit (not shown). The refrigerant circuit is filled with a refrigerant which circulates to perform a refrigeration cycle. The heat exchanger (16) functions as an evaporator which cools the air with a low-pressure refrigerant flowing in the heat exchanger. The heat exchanger also functions as a condenser which heats the air with a high-pressure refrigerant flowing in the heat exchanger. The fan (17) transfers the air in the air passage (11a).

### <Configuration for Discharge Unit>

The discharge unit (20) is configured as a streamer discharge unit. That is, the discharge unit (20) performs a streamer discharge, thereby generating low-temperature plasma, which generates highly reactive active species (e.g., high-speed electrons, ions, radicals and ozone) in the air. The discharge unit (20) includes a casing (21), a voltage supply section (30) housed in the casing (21), and a discharge process section (40) housed in the casing (21).

### [Casing]

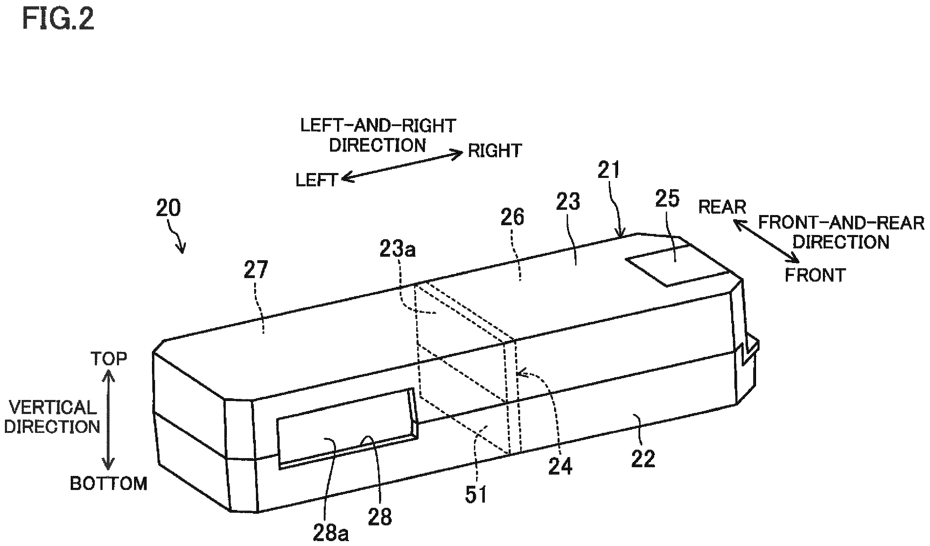

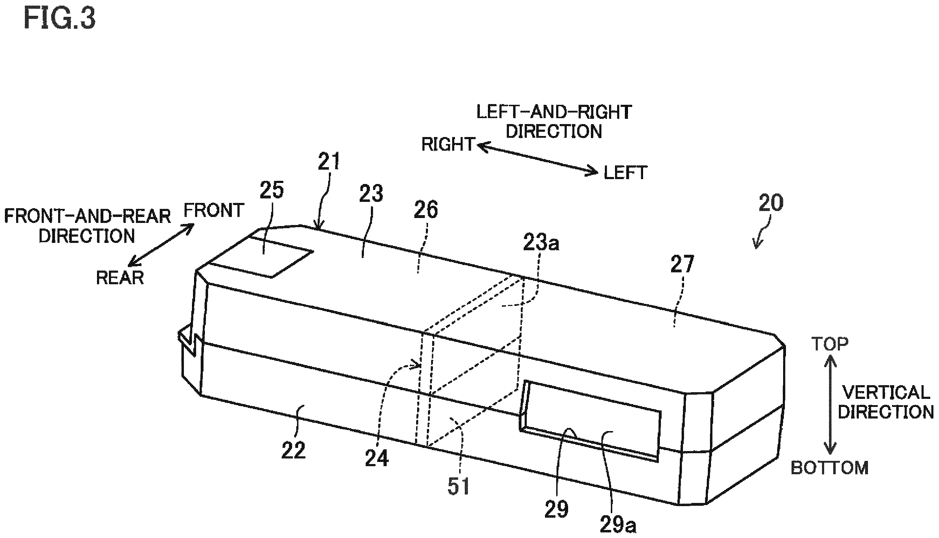

As illustrated in FIG. 2 and FIG. 3, the casing (21) has a horizontally-oriented, generally parallelepiped box-like shape. The casing (21) is made of an insulating resin material. The casing (21) includes a lower case (22) and an upper case (23) attached to the upper side of the lower case (22). A partition (24) is provided in the casing (21) at a longitudinal middle portion (i.e., a middle portion in the left-and-right direction) of the casing (21). The partition (24) divides the interior of the casing (21) into two spaces (i.e., left and right spaces). The right space constitutes a housing chamber (26), and the left space constitutes a processing chamber (27) (i.e., an air passage).

The partition (24) is comprised of an upper partition wall (23a) and a lower partition wall (51). The upper partition wall (23a) is an integrally-formed wall in the upper case (23). The lower partition wall (51) is formed integrally with an insulating member (41), which will be described later. The upper partition wall (23a) and the lower partition wall (51) of the partition (24) are arranged one above the other such that the lower surface of the upper partition wall (23a) and the upper surface of the lower partition wall (51) are adjacent to each other.

As illustrated in FIG. 2, the front surface of the casing (21) is provided with a first air hole (28) (i.e., an inlet hole). The first air hole (28) is positioned closer to the left side of the casing (21) so that the hole may communicate with the processing chamber (27). A first shielding plate (28a) is provided behind the first air hole (28). The first shielding plate (28a) has a rectangular shape generally larger in size than the opening area of the first air hole (28). The first shielding plate (28a) serves as a shielding member which avoids exposure of the discharge process section (40) (namely, discharge needles (73, 74) of the discharge electrode (70), etc.) to the outside of the casing (21). A gap (i.e., an air inflow path) is formed between the edge of the first air hole (28) and the first shielding plate (28a). The air which has flowed in through the first air hole (28) passes through this gap to flow into the interior of the processing chamber (27).

As illustrated in FIG. 3, the rear surface of the casing (21) is provided with a second air hole (29) (i.e., an outflow hole (29)). The second air hole (29) is positioned closer to the left side of the casing (21) so that the hole may communicate with the processing chamber (27). A second shielding plate (29a) is provided behind the second air hole (29). The second shielding plate (29a) has a rectangular shape generally larger in size than the opening area of the second air hole (29). The second shielding plate (29a) serves as a shielding member which avoids exposure of the discharge process section (40) (namely, the discharge needles (73, 74) of the discharge electrode (70), etc.) to the outside of the casing (21). A gap (i.e., an air intake path) is formed between the edge of the second air hole (29) and the second shielding plate (29a). The air in the processing chamber (27) passes through this gap to flow out of the casing (21).

The first shielding plate (28a) and the second shielding plate (29a) are integrally formed with the casing (21). The first shielding plate (28a) and the second shielding plate (29a) are made of a resin material with a higher flame-resistance than the material forming the casing (21).

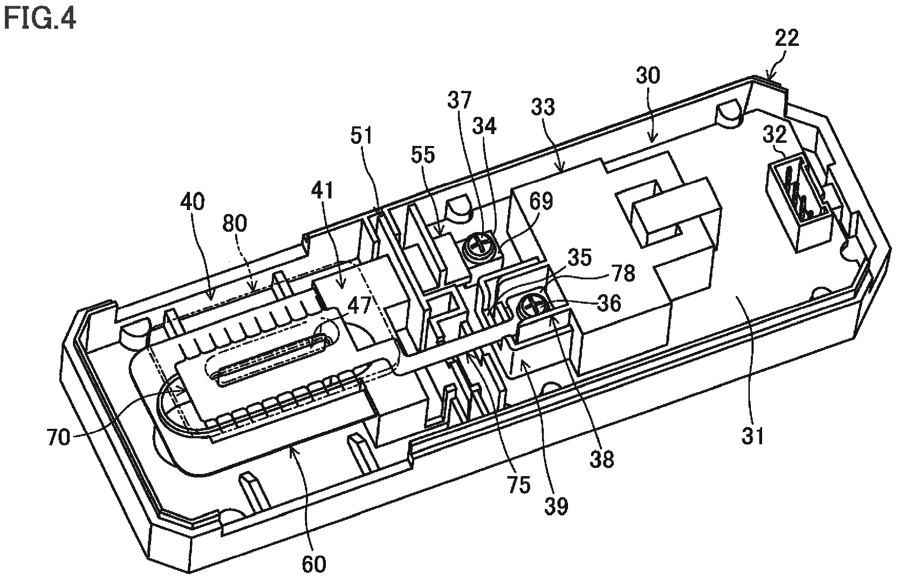

As illustrated in FIG. 2 and FIG. 3, a slidable cover (25) is provided on the right end of the upper case (23) at a middle portion in the front-and-rear direction. The slidable cover (25) may be attached to, or detached from, the body of the casing (21). If the slidable cover (25) is detached, a connector (32) (see FIG. 4) of the voltage supply section (30) is exposed to the outside of the casing (21).

### [Voltage Supply Section]

As illustrated in FIG. 4, the voltage supply section (30) is located in the housing chamber (26). The voltage supply section (30) is configured to supply source voltage supplied from an external power source to the discharge process section (40). The voltage supply section (30) has a substrate (31), a connector (32), a power transformer (33), and an earth terminal (34). The substrate (31) is arranged near the bottom of the housing chamber (26). The substrate (31) has a plate-like shape elongated in the right-and-left direction and is arranged to lie across the entire region of the housing chamber (26).

The connector (32) is arranged on the upper surface of a right end portion of the substrate (31). The connector (32) is exposed to the outside of the casing (21) when the above-described slidable cover (25) is detached. A wire electrically connected to the external power source is connected to the connector (32).

The power transformer (33) is arranged on the upper surface of the substrate (31) at a location closer to the right side of the substrate (31). The power transformer (33) is configured to boost the voltage supplied to the power transformer (33) via the connector (32). A feed terminal (35) is provided at a left end portion of the power transformer (33). A feed plate (75) of the discharge electrode (70) is fixed to the feed terminal (35) with a fastening member (i.e., a screw (36)).

The earth terminal (34) is arranged on the upper surface of the substrate (31) at a location closer to the left end and the rear end of the substrate (31). An earth plate (68) of the counter electrode (60) is fixed to the earth terminal (34) with a fastening member (i.e., a screw (37)).

The feed terminal (35) is located at a relatively higher position than the earth terminal (34). In other words, the feed terminal (35) and the earth terminal (34) are at different positions in the vertical direction. The feed terminal (35) is located closer to the processing chamber (27) than the earth terminal (34) is. In other words, the feed terminal (35) and the earth terminal (34) are at different positions in the left-and-right direction (i.e., in the longitudinal direction of the discharge electrode (70) or the counter electrode (60)). Further, the feed terminal (35) is located closer to the front side than the earth terminal (34) is. In other words, the feed terminal (35) and the earth terminal (34) are at different positions in the front-and-rear direction. This structure increases the distance between the feed terminal (35) and the earth terminal (34) and thus contributes to an increase in a creepage distance between the feed terminal (35) and the earth terminal (34).

The power transformer (33) has an inner wall portion (38) which surrounds the feed terminal (35). The inner wall portion (38) is made of an insulating resin material of which the upper side and the left side are open. The inner wall portion (38) has a cross-section having a square U shape whose left side is open (i.e., an inverted C shape).

The inner wall portion (38) is surrounded by an outer wall portion (39). The outer wall portion (39) is made of an insulating resin material of which the upper side and the right side are open. The outer wall portion (39) has a cross-section having a square U shape whose right side is open (i.e., a C shape). There is a gap between the inner wall portion (38) and the outer wall portion (39) along the entire periphery.

The provision of the inner wall portion (38) and the outer wall portion (39) surrounding the feed terminal (35) contributes to a further increase in the creepage distance between the feed terminal (35) and the earth terminal (34).

### [Discharge Process Section]

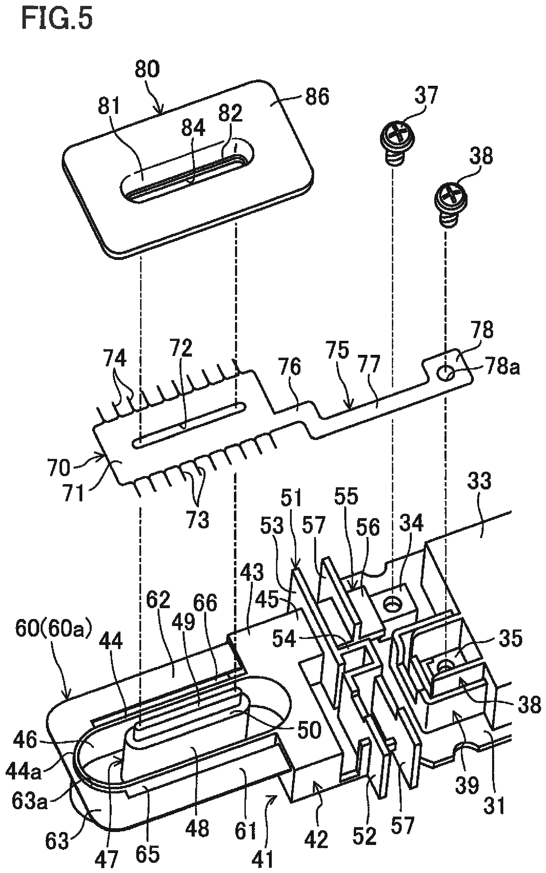

As illustrated in FIG. 4 and FIG. 5, the discharge process section (40) is generally located in the processing chamber (27). The discharge process section (40) is configured to generate a streamer discharge to purify air. The discharge process section (40) includes an insulating member (41), a counter electrode (60), a discharge electrode (70), and a stabilizer (80).

The insulating member (41) is made of an insulating resin material and serves as a support member which supports the discharge electrode (70) and the counter electrode (60) while insulating these electrodes from each other. The counter electrode (60) and the discharge electrode (70) are made of a conductive metal material. The counter electrode (60) is electrically connected to an earth connector (69) and is grounded. The discharge electrode (70) is electrically connected to the voltage supply section (30), and receives a high voltage (e.g., 7.0 kV). When a voltage is supplied to the discharge electrode (70) from the voltage supply section (30), a streamer discharge is generated between the two electrodes (60, 70). The stabilizer (80) is made of a conductive resin material, and has the same electric potential as the discharge electrode (70). The stabilizer (80) serves as a conductive member (i.e., a fixing member) for creating a stable electric field near the discharge electrode (70).

### [Insulating Member]

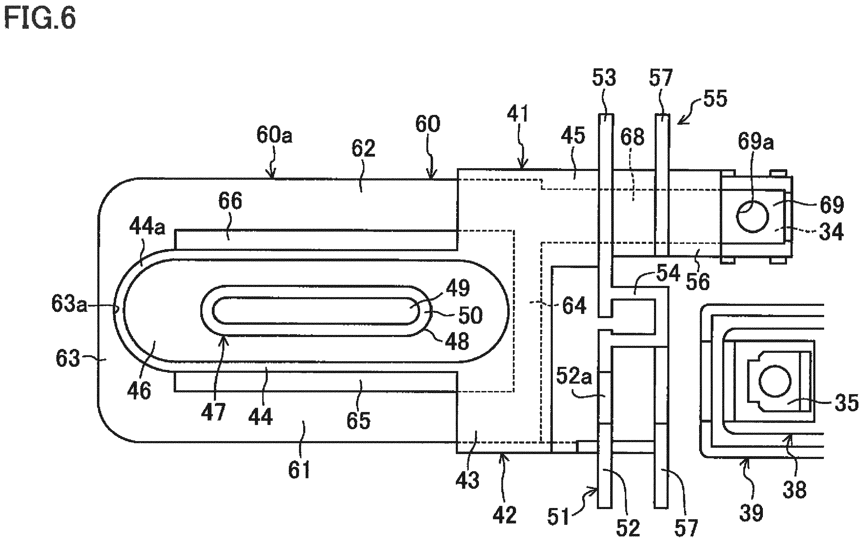

As illustrated in FIG. 4, the insulating member (41) is placed on the bottom of the lower case (22). As is also illustrated in FIG. 5, FIG. 6 and FIG. 9, the insulating member (41) includes an embedding portion (42), a base (44), a support (47), a lower partition wall (51), and a creepage distance increase portion (55).

The embedding portion (42) is arranged in the processing chamber (27) at a location on the left side of the lower partition wall (51). The embedding portion (42) has a body (43) and a connecting portion (45). The body (43) has a rectangular parallelepiped shape extending between the front edge and the rear edge of the lower case (22). The connecting portion (45) is formed continuously between a rear end portion of the right side surface of the body (43) and the lower partition wall (51).

The base (44) protrudes to the left from a middle portion, in the front-and-rear direction, of the left side surface of the body (43). The base (44) includes an arc portion (44a) at its end which has an arc-shaped cross section. The insulating member (41) is provided with an oblong groove (46) (i.e., a recess) extending from the base (44) to a middle portion of the body (43). The oblong groove (46) is a cylindrical hole having an oblong shape elongated in the left-and-right direction, with a closed bottom and an open top.

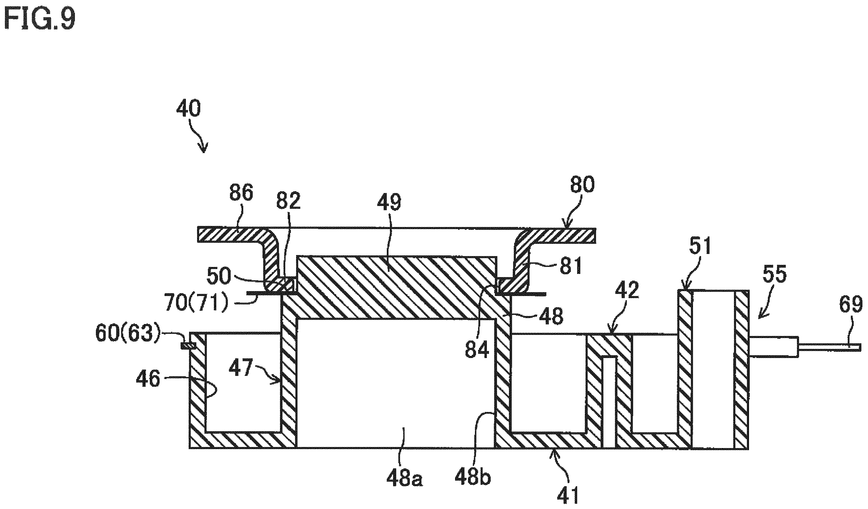

The support (47) is located at a middle portion, in the left-and-right direction and the front-and-rear direction, of the oblong groove (46). The support (47) includes a support body (48) and a projection (49) (i.e., a fitted portion) which projects upward from the support body (48). The support body (48) has a columnar shape having an oblong transverse cross-section elongated in the left-and-right direction. The support body (48) has a hollow (48a) therein which extends upward from the lower end of the support body (48) (see FIG. 9). The support body (48) has an inner periphery surface (48b) which forms an oblong shape elongated in the left-and-right direction to surround the hollow (48a).

The projection (49) is located at a middle portion, in the left-and-right direction and the front-and-rear direction, of the support body (48). Similarly to the support body (48), the projection (49) has a columnar shape having an oblong cross-section elongated in the left-and-right direction. The projection (49) has a height, a width in the left-and-right direction, and a thickness in the front-and-rear direction, all of which are less than those of the support body (48). Thus, a ring-shaped mounting surface (50) that is oblong in the left-and-right direction is formed on the upper end surface of the support body (48) so as to surround the projection (49). The mounting surface (50) is approximately level and flat. The support (47) supports the discharge electrode (70) and the stabilizer (80) which will be described in detail later.

The lower partition wall (51) extends between the front edge and the rear edge of the lower case (22). The lower partition wall (51) includes a first lateral wall (52) positioned closer to the front side of the lower case (22), a second lateral wall (53) positioned closer to the rear side of the lower case (22), and a protruding wall (54) which protrudes to the right from between the lateral walls (52, 53). The first lateral wall (52) and the second lateral wall (53) have a plate-like shape extending in the front-and-rear direction. The protruding wall (54) has a cross-section having a square U shape whose left side is open (i.e., an inverted C shape) and is continuous with a rear portion of the first lateral wall (52) and a front portion of the second lateral wall (53). A vertically-oriented gap is formed between the first lateral wall (52) and the second lateral wall (53), and this gap communicates with the space surrounded by the protruding wall (54).

The creepage distance increase portion (55) is configured of a plurality of horizontal plates (56) and a plurality of vertical plates (57) assembled to intersect with each other. Some of these plates are continuous with the lower partition wall (51). As such, the lower partition wall (51) and the creepage distance increase portion (55) form a so-called labyrinth structure, in which a plurality of insulating plates having vertical or horizontal surfaces are complicatedly assembled to one another. As a result, the creepage distance between the discharge electrode (70) and the counter electrode (60) is increased.

### [Counter Electrode]

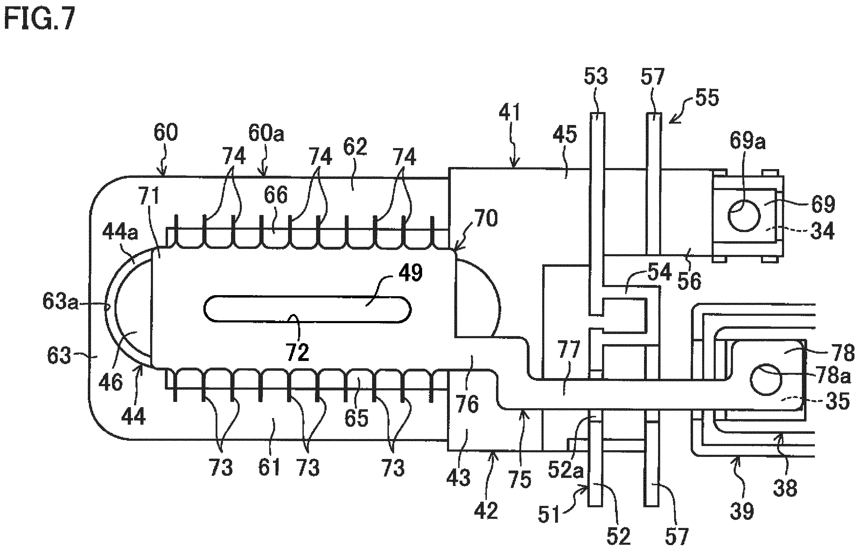

As illustrated in FIG. 4 to FIG. 7 and FIG. 9, the counter electrode (60) is integrally formed with the insulating member (41). Specifically, the counter electrode (60) and the insulating member (41) are configured as an integrally-formed unit formed by insert molding. The counter electrode (60) has a flat plate-like shape so that the entire portion thereof may lie on the same plane (i.e., on a horizontal plane). The counter electrode (60) includes a rectangular frame-like counter electrode body (60a) and an earth plate (68) extending to the right from a rear portion of the right side of the counter electrode body (60a).

The counter electrode body (60a) is comprised of a first counter plate (61), a second counter plate (62), a first connecting plate (63), and a second connecting plate (64) which are assembled to one another. The first counter plate (61) is positioned closer to the front of the counter electrode body (60a) and extends in the left-and-right direction. The second counter plate (62) is positioned closer to the rear of the counter electrode body (60a) and extends in the left-and-right direction. A first gap (65) having a rectangular shape elongated in the left-and-right direction is formed between the first counter plate (61) and the front side of the base (44). A second gap (66) having a rectangular shape elongated in the left-and-right direction is formed between the second counter plate (62) and the rear side of the base (44).

The first connecting plate (63) is positioned closer to the left of the counter electrode body (60a) and extends in the front-and-rear direction. The first connecting plate (63) connects the left end of the first counter plate (61) with the left end of the second counter plate (62). Formed at an inner periphery (i.e., the right side) of the first connecting plate (63) is an arc groove (63a) to which the arc portion (44a) of the base (44) is fitted. The second connecting plate (64) is positioned closer to the right of the counter electrode body (60a) and extends in the front-and-rear direction. The second connecting plate (64) connects the right end of the first counter plate (61) with the right end of the second counter plate (62). The second connecting plate (64) is embedded in an upper portion of the base (43).

Almost the entire portion of the earth plate (68) is embedded in the embedding portion (42) of the insulating member (41) and in between the horizontal plates (56). The earth plate (68) has a tip end which protrudes further to the right from the horizontal plate (56) and is exposed to the outside of the insulating member (41). The tip end of the earth plate (68) serves as the earth connector (69). The earth connector (69) has an approximately square plate-like shape and is provided with an insertion hole (69a) through the center of which a screw (37) is inserted. The earth connector (69) is placed to be layered on the upper surface of the earth terminal (34) and is connected to the earth terminal (34) with the screw (37).

### [Discharge Electrode]

As illustrated in FIG. 4, FIG. 5, FIG. 7 to FIG. 9, and FIG. 11, the discharge electrode (70) is arranged above the insulating member (41). The discharge electrode (70) has a thin plate-like shape so that the entire portion thereof may lie on the same plane (i.e., on a horizontal plane). The discharge electrode (70) is much thinner than the counter electrode (60). The discharge electrode (70) includes an electrode support plate (71), a plurality of discharge needles (73, 74) (i.e., discharge sections) supported on the side edges of the electrode support plate (71), and a feed plate (75) protruding to the right from a front end portion of the right side of the electrode support plate (71).

The electrode support plate (71) is arranged above the base (44). The electrode support plate (71) extends in the left-and-right direction along the base (44). A positioning hole (72) (i.e., an opening), into which the projection (49) of the support (47) is fitted, is formed at the center of the electrode support plate (71) (i.e., a middle portion in the longitudinal direction and width direction of the electrode support plate (71)). The positioning hole (72) has an oblong shape elongated in the left-and-right direction to correspond to the profile of the projection (49). Once the projection (49) is fitted in the positioning hole (72), the electrode support plate (71) is brought on the mounting surface (50). As a result, the flatness of the electrode support plate (71) is maintained. In other words, the electrode support plate (71) is kept flat by being supported on the mounting surface (50). A relative positional relationship between the discharge electrode (70) and the insulating member (41) is also determined, which in turn determines a relative positional relationship between the discharge electrode (70) and the counter electrode (60).

In the state in which the projection (49) is fitted in the positioning hole (72), a slight gap (not shown) is left between longitudinal end portions (i.e., left and right end portions) of the projection (49) and the inner periphery of the positioning hole (72). That is, the positioning hole (72) has a longer maximum longitudinal length than the projection (49). This slight gap allows the projection (49) to thermally expand (i.e., extend under heat) in its longitudinal direction in the positioning hole (72). Thus, stress may be prevented from being concentrated at both longitudinal ends of the inner periphery of the positioning hole (72) when the thermal expansion of the projection (49) occurs.

The plurality of first discharge needles (73), each in the form of a thin needle or rod, are supported on the front edge of the electrode support plate (71). The plurality of first discharge needles (73) are arranged at regular intervals along the front edge of the electrode support plate (71) and extend horizontally straight forward from the electrode support plate (71). The first discharge needles (73) are arranged parallel to one another. The plurality of second discharge needles (74), each in the form of a thin needle or rod, are supported on the rear edge of the electrode support plate (71). The plurality of second discharge needles (74) are arranged at regular intervals along the rear edge of the electrode support plate (71) and extend horizontally straight rearward. The second discharge needles (74) are arranged parallel to one another. The electrode support plate (71) extends in a direction in which the plurality of discharge needles (73, 74) are arranged. As such, a large number of discharge needles (73, 74) may be provided at the front and rear side edges of the electrode support plate (71).

The discharge electrode (70) of the present embodiment is provided with ten first discharge needles (73) and ten second discharge needles (74). The number of these discharge needles (73, 74) is merely an example. The first discharge needle (73) and the second discharge needle (74) are generally coaxial with each other in the front-and-rear direction. The first discharge needle (73) and the second discharge needle (74) may be misaligned with each other in the left-and-right direction.

The first discharge needles (73) are parallel to the first counter plate (61). The second discharge needles (74) are parallel to the second counter plate (62). A lower portion of the tip of each first discharge needle (73) faces the first counter plate (61), and a lower portion of the tip of the second discharge needle (74) faces the second counter plate (62).

The feed plate (75) includes, from left to right, a first feed portion (76), a second feed portion (77), and a feed connecting portion (78). The first feed portion (76) protrudes to the right from a front edge portion of the right side of the electrode support plate (71). The second feed portion (77) is connected to a front edge portion of the tip of the first feed portion (76) and protrudes to the right. A portion of the second feed portion (77) is supported on the bottom of a recessed groove (52a) of the first lateral wall (52). The tip end of the second feed portion (77) serves as the feed connecting portion (78). The feed connecting portion (78) has an approximately square plate-like shape and is provided with an insertion hole (78a) through the center of which a screw (36) is inserted. The feed connecting portion (78) is placed to be layered on the upper surface of the feed terminal (35) and is connected to the feed terminal (35) with the screw (36).

### [Stabilizer]

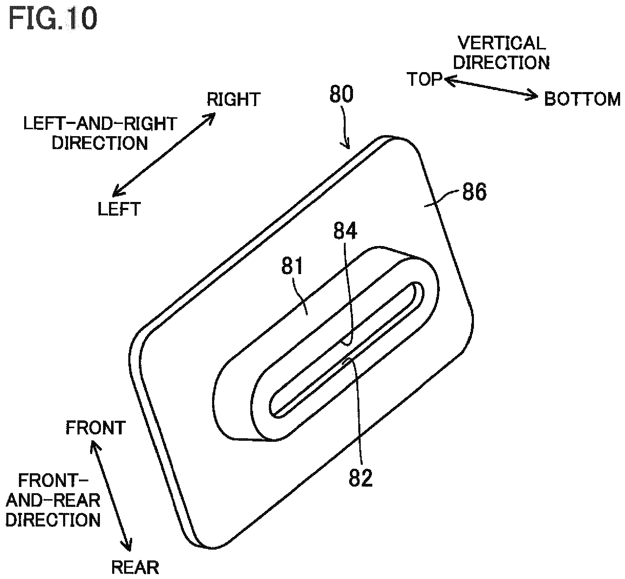

The stabilizer (80) is arranged above the support (47) and the discharge electrode (70). As illustrated in FIG. 5 and FIG. 8 to FIG. 11, the stabilizer (80) includes a cylindrical wall portion (81) and a flanged portion (86) projecting out from an upper end portion of the cylindrical wall portion (81) to all the directions.

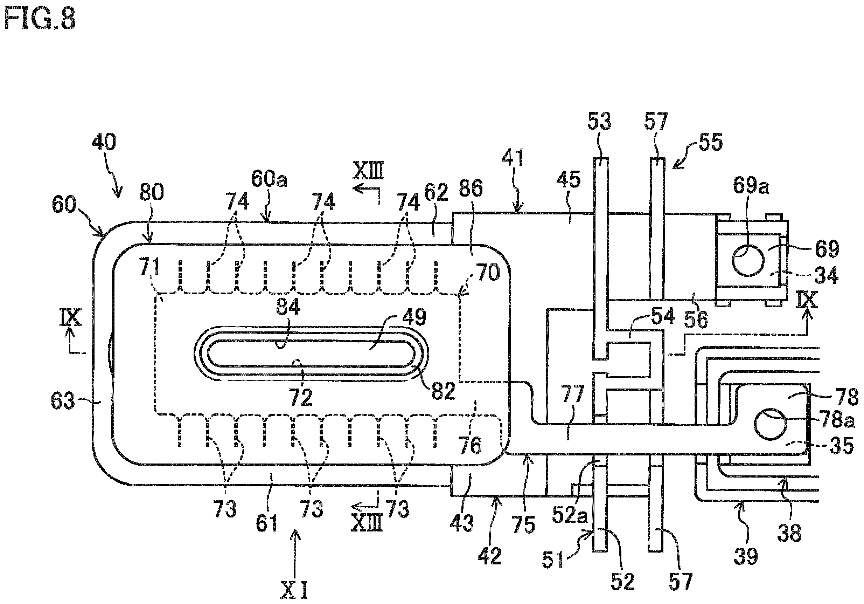

The cylindrical wall portion (81) is in a cylindrical shape having an oblong cross-section elongated in the left-and-right direction. A lower edge portion of the inner periphery of the cylindrical wall portion (81) is provided with an oblong annular projection (82) (see FIG. 5 and FIG. 8). The annular projection (82) projects inward from an inner peripheral surface of the lower edge of the cylindrical wall portion (81). The bottom surface of the cylindrical wall portion (81) and the bottom surface of the annular projection (82) are flush with each other and form a horizontal plane.

An oblong opening (84) elongated in the left-and-right direction is formed by being surrounded by the annular projection (82). The projection (49) of the insulating member (41) is fitted into the oblong opening (84). As a result, the stabilizer (80) is arranged above the electrode support plate (71), and a relative positional relationship among the stabilizer (80), the electrode support plate (71), and the counter electrode (60) is determined. In the state in which the projection (49) is fitted in the oblong opening (84), a gap is left between the projection (49) and the inner peripheral surface of the cylindrical wall portion (81).

The profile of the flanged portion (86) has a rectangular plate-like shape elongated in the left-and-right direction. In the state in which the projection (49) is fitted in the cylindrical wall portion (81), the flanged portion (86) is approximately horizontal. The flanged portion (86) projects forward such that its front edge is positioned forward of the tips of the first discharge needles (73). The flanged portion (86) projects rearward such that its rear edge is positioned rearward of the tips of the second discharge needles (74). In other words, the flanged portion (86) covers the entire portions of the first discharge needles (73) and the second discharge needles (74) from above. The bottom surface of the flanged portion (86) forms a horizontal plane and is parallel with the respective discharge needles (73, 74) along the discharge needles (73, 74).

### -Operation-

Operation of the air conditioner (10) will be described. The air conditioner (10) illustrated in FIG. 1 switches between a cooling operation and a heating operation. When the fan (17) of the air conditioner (10) is actuated, air in the indoor space (S) is drawn into the air passage (11a) through the room-air duct (12). This air passes through the prefilter (14). The prefilter (14) catches relatively-large dust contained in the air.

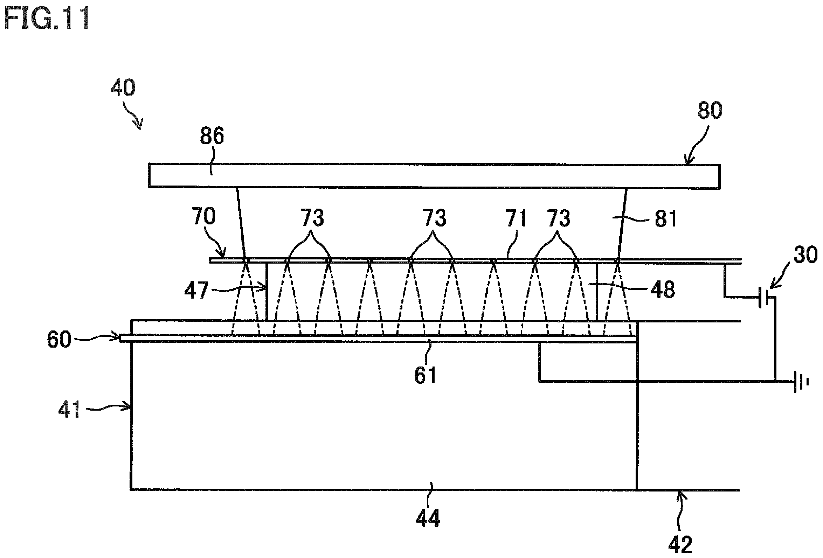

The air which has passed through the prefilter (14) passes through the discharge unit (20) (see FIG. 2). Specifically, the air flows into the processing chamber (27) through the first air hole (28) of the casing (21). In the discharge unit (20), a high voltage is supplied from the power transformer (33) of the voltage supply section (30) to the discharge electrode (70). As a result, a streamer discharge develops from the tip of each discharge needle (73, 74) of the discharge electrode (70) to the counter plates (61, 62) (see FIG. 11). The high voltage is also supplied to the stabilizer (80) connected to the discharge electrode (70). Thus, the streamer discharges emerged from the discharge needles (73, 74) to the counter plates (61, 62) are stabilized.

When a streamer discharge is generated in the discharge process section (40), active species are generated in the air due to the streamer discharge. These active species oxidize and decompose harmful or odor components in the air, thereby purifying the air. The air in the processing chamber (27) flows out of the casing (21) from the second air hole (29) together with the active species (see FIG. 3) and passes through the catalyst filter (15). The catalyst filter (15) adsorbs odor components in the air. The adsorbed odor components are decomposed by the active species, thereby regenerating the adsorbent.

The air purified in this manner is heated or cooled by the heat exchanger (16) and is then supplied into the indoor space (S) through the air-supply duct (13). As a result, the indoor space (S) is heated or cooled, and the indoor air is purified.

### <Fabrication Process of Discharge Unit>

Now, a fabrication process of the discharge unit (20) will be described. The fabrication process includes a process for fabricating the discharge process section (40) and a placement process for mounting the discharge process section (40) and the voltage supply section (30) into the casing (21).

### <Fabrication Process of Discharge Process Section>

The fabrication process of the discharge process section (40) will be described with reference to FIG. 5. In the fabrication process of the discharge process section (40), the insulating member (41), the discharge electrode (70), and the stabilizer (80) are assembled to one another sequentially from bottom to top.

In the first step, the insulating member (41) and the counter electrode (60) are formed as an integrally-formed unit by insert molding.

In the second step, the projection (49) of the insulating member (41) is fitted in the positioning hole (72) of the discharge electrode (70). As a result, the electrode support plate (71) is arranged on the mounting surface (50), thereby achieving positioning and temporary fixing of the discharge electrode (70).

In the third step, the projection (49) of the insulating member (41) is fitted into the cylindrical wall portion (81) of the stabilizer (80). As a result, the cylindrical wall portion (81) of the stabilizer (80) is arranged above the electrode support plate (71). In this state, the electrode support plate (71) is sandwiched between the mounting surface (50) and the cylindrical wall portion (81).

In the fourth step, the projection (49) is fixed to the stabilizer (80) and the electrode support plate (71). Specifically, this fixing is achieved by ultrasonic wave welding. That is, the tip of the projection (49) is melted by an ultrasonic wave, and the melted resin flows into a space above the annular projection (82) and a gap around the oblong opening (84). When the melted resin solidifies, the stabilizer (80) and the projection (49), as well as the projection (49) and the electrode support plate (71), are fixed to each other. As a result, the discharge electrode (70) is fixed or supported between the stabilizer (80) and the insulating member (41).

In the fourth step, the stabilizer (80) and the projection (49) do not necessarily have to be fixed to each other by the ultrasonic wave welding, but may be fixed by other techniques, such as thermal welding, vibration welding, and bonding.

### <Placement Process>

Now, a process for assembling the discharge process section (40) and the voltage supply section (30) into the casing (21) will be described. In this placement process, all the elements are assembled from above the lower case (22).

First, the voltage supply section (30) is assembled to the bottom of the top-open lower case (22) closer to the right (i.e., the bottom of the housing chamber (26)).

Next, the discharge process section (40) built in the above-described manner is assembled to the bottom of the lower case (22) closer to the left (i.e., the bottom of the processing chamber (27)). In this assembly, as illustrated in FIG. 4, the earth connector (69) is connected to the earth terminal (34) with the screw (37), and the feed connecting portion (78) is connected to the feed terminal (35) with the screw (36). It is therefore possible to supply a voltage from the voltage supply section (30) to the discharge electrode (70).

Then, the upper case (23) is assembled on top of the lower case (22). The discharge unit (20) illustrated in FIG. 2 and FIG 3 is obtained in this manner.

### <Detailed Configuration and Advantages of Discharge Electrode, Insulating Member, and Stabilized

Examples of the structure in which the insulating member (41) supports the electrode support plate (71) of the discharge electrode (70) may include a structure in which the insulating member (41) supports both longitudinal ends of the electrode support plate (71). This structure may insulate the discharge electrode (70) and the counter electrode (60) from each other, while determining relative positions between the discharge electrode (70) and the counter electrode (60). However, this structure may cause a problem in which if an ambient temperature of the discharge unit (20) changes during the fabrication or use of the discharge unit (20), the electrode support plate (71) may warp and a suitable distance between the discharge needles (73, 74) and the counter electrode (60) may not be maintained.

This is because the resin insulating member (41) has a thermal expansion coefficient greater than the metal discharge electrode (70). Thus, for example, if an ambient temperature of the discharge unit (20) drops significantly, the thermal contract of the insulating member (41) in a longitudinal direction may become greater than the thermal contract of the electrode support plate (71) in a longitudinal direction, and the electrode support plate (71) may warp in a thickness direction. The warpage of the electrode support plate (71) in the thickness direction may result in change in the distance particularly between the discharge needles (73, 74) near a longitudinal middle portion of the electrode support plate (71) and the counter electrode (60), which may prohibit continuous generation of optimum streamer discharges.

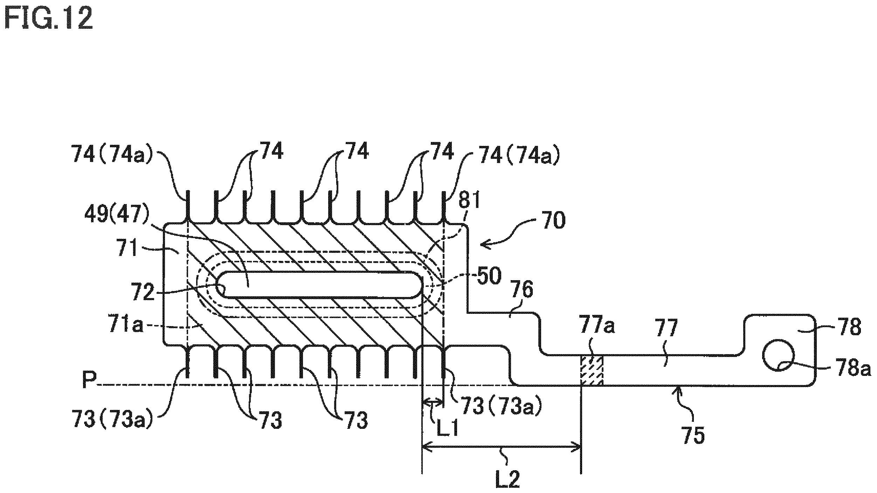

To address this problem, the support (47) of the insulating member (41) in the present embodiment supports a middle region (71a) of the electrode support plate (71). As illustrated in FIG 12, the middle region (71a) is part of the electrode support plate (71) between two discharge needles (73a, 74a) of the plurality of discharge needles (73, 74) arranged on both longitudinal ends of the electrode support plate (71) (i.e., a hatched region of the electrode support plate (71) in FIG. 12). In other words, the support (47) of the insulating member (41) does not support both end portions of the electrode support plate (71), but supports the middle region of the electrode support plate (71). If the support (47) supports both the end portions of the electrode support plate (71), the electrode support plate (71) may warp easily due to ambient temperature changes as mentioned above. On the other hand, the support (47) of the present embodiment supports the middle region (71a) of the electrode support plate (71). Thus, even if thermal shrinkage of the insulating member (41) occurs, the middle region (71a) of the electrode support plate (71) does not warp severely. Accordingly, even if the ambient temperature changes, the distance between the discharge needles (73, 74) and the counter electrode (60) does not change much.

The electrode support plate (71) has a rectangular plate-like shape extending in a direction in which the plurality of discharge needles (73, 74) are arranged. As such, a large number of discharge needles (73, 74) may be provided at the front and rear side edges of the electrode support plate (71), which increases the amount of active species generated by streamer discharges. Although the electrode support plate (71) in the shape extending in the arrangement direction of the discharge needles (73, 74) may warp easily, such warpage may be prevented by the support (47) of the insulating member (41).

The support (47) of the insulating member (41) supports a longitudinal middle portion of the electrode support plate (71). In other words, the insulating member (41) supports a portion of the electrode support plate (71) near the center of mass of the electrode support plate (71), thereby making it possible to stably support the electrode support plate (71).

The middle region (71a) of the electrode support plate (71) is supported by being sandwiched between the insulating member (41) and the stabilizer (80). Thus, the discharge electrode (70) may be supported by a relatively simple structure. In this structure, almost no load acts on the discharge needles (73, 74). The discharge needles (73, 74) are thus prevented from being bent or broken in the process of forming the discharge needles and may generate stable streamer discharges.

The flanged portion (86) is opposed to the counter electrode (60) with the discharge needles (73, 74) sandwiched therebetween. The flanged portion (86) has the same electric potential as the discharge needles (73, 74), and extends along the discharge needles (73, 74). The flanged portion (86) of the conductive member (80) may stabilize the discharges directed to the counter electrode (60) from the discharge needles (73, 74). The middle region (71a) of the electrode support plate (71) is supported by being sandwiched between the conductive member (80) and the insulating member (41). That is, the conductive member (80) serves as a member which stabilizes discharges and also as a fixing member which stably supports the discharge electrode (70).

The projection (49) of the support (47) is fitted in the positioning hole (72) in the middle region (71a) of the electrode support plate (71). This structure provides reliable positioning between the electrode support plate (71) and the support (47), and hence between the discharge electrode (70) and the counter electrode (60).

The positioning hole (72) of the middle region (71a) has an elongated shape extending in the arrangement direction of the discharge needles (73, 74). The projection (49) of the insulating member (41) has an elongated shape corresponding to this shape of the positioning hole (72). Such a structure in which a portion of the middle region (71a) supported by the projection (49) has an increased dimension in the longitudinal direction of the electrode support plate (71), may effectively prevent warpage of the middle region (71a) of the electrode support plate (71).

The support (47) of the insulating member (41) includes a support body (48) and a projection (49) which protrudes from the upper end of the support body (48). The projection (49) is fitted in the positioning hole (72) of the electrode support plate (71), thereby having the middle region (71a) of the electrode support plate (71) supported on the insulating member (41). The insulating member (41) is provided with a ring-shaped mounting surface (50) surrounding the projection (49) on the upper end of the support body (48). The electrode support plate (71) is placed on the mounting surface (50). The mounting surface (50) is a planar surface which contributes to maintaining the flatness of the electrode support plate (71). Hence, the mounting surface (50) may keep an optimum distance between the plurality of discharge needles (73, 74) and the counter electrode (60).

The projection (49) of the insulating member (41) is fitted in the cylindrical wall portion (81) of the conductive member (80). Thus, relative positions among the insulating member (41), the discharge electrode (70), and the conductive member (80) are determined, which in turn determines relative positions between the discharge needles (73, 74) and the counter electrode (60), and between the discharge needles (73, 74) and the flanged portion (86). The electrode support plate (71) is supported by being sandwiched between the insulating member (41) and the cylindrical wall portion (81).

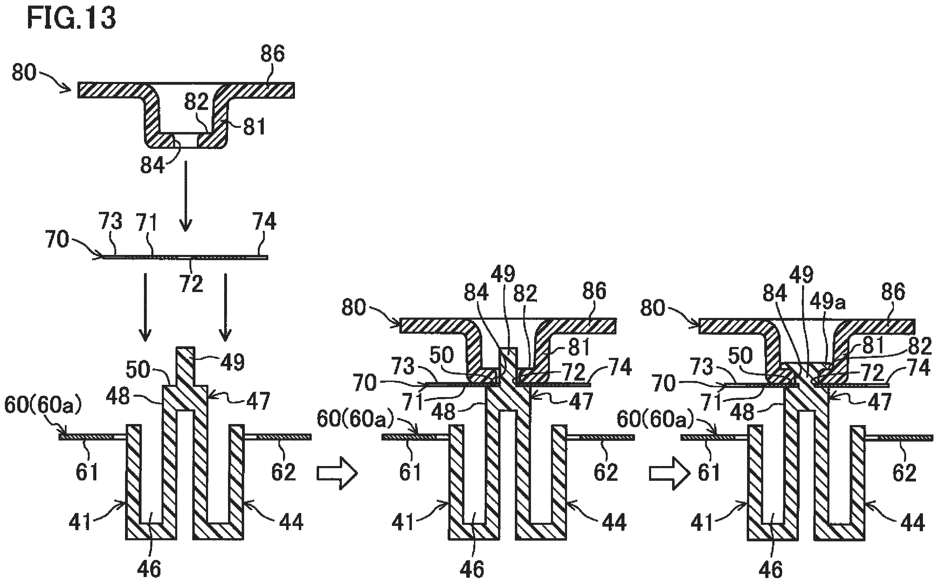

As illustrated in FIG. 13, the discharge process section (40) is assembled by inserting the projection (49) of the insulating member (41) in the positioning hole (72) of the discharge electrode (70), and thereafter inserting this projection (49) in the oblong opening (84) of the cylindrical wall portion (81). The tip of the projection (49) is melted in this state by an ultrasonic wave, and the melted resin flows into a space between the projection (49) and the cylindrical wall portion (81). The melted resin reaches a region close to an inner periphery of the positioning hole (72) of the electrode support plate (71). When the melted resin solidifies, a solidified portion (49a) having a larger diameter than the projection (49) is formed on the annular projection (82) in the space surrounded by the cylindrical wall portion (81). As a result, the stabilizer (80) and the insulating member (41) are firmly fixed to each other, and the discharge electrode (70) is sandwiched between the cylindrical wall portion (81) and the mounting surface (50) without a gap therebetween. Accordingly, the discharge needles (73, 74) of the discharge electrode (70), the stabilizer (80), and the counter electrode (60) may be kept at desired distances from one another in the vertical direction.

Further, as illustrated in FIG. 12, it is recommended that the dimensions of the discharge electrode (70) be determined as follows. First, it is recommended that the length L1 extending from one of the two longitudinal ends, which is closer to the feed plate (75), of the positioning hole (72) of the electrode support plate (71) to the discharge needle (73a) closest to the feed plate (75) be 2 mm or less. If the length L1 is greater than 2 mm, a portion of the electrode support plate (71) corresponding to this length may warp. Further, as illustrated in FIG. 12, it is recommended that the length L2 extending from one of the two longitudinal ends, which is closer to the feed plate (75), of the positioning hole (72) of the electrode support plate (71) to a fixed end (77a) of the feed plate (75) (i.e., the portion between the upper partition wall (23a) and the lower partition wall (51)) be 8 mm or less. This structure may prevent the warpage of the portion of the discharge electrode (70) corresponding to this length, too.

As illustrated in FIG. 12, the discharge electrode (70) is configured such that the tips (i.e., front tips) of the first discharge needles (73, 74) are located closer to the electrode support plate (71) than the phantom line (i.e., the phantom line P in FIG. 12) extending in the left-and right direction along the front edge of the second feed portion (77) of the feed plate (75) is. This structure prevents the tips of the first discharge needles (73) from protruding forward of the earth plate (75) and thus prevents the discharge needles (73) from coming into contact with any objects and being bent by such a contact with the objects in the process of e.g. forming the discharge needles.

### -Advantages of Embodiment-

In the above embodiment, the insulating member (41) supports the middle region (71a) of the electrode support plate (71), which may reduce the risk of warpage of the electrode support plate (71) due to temperature changes. This structure may maintain an optimum distance between the discharge needles (73, 74) and the counter electrode (60), and achieve continuous generation of desired stable discharges.

The electrode support plate (71) has a rectangular plate-like shape extending in the left-and-right direction. Thus, a large number of discharge needles (73, 74) may be supported on both side edge portions of the electrode support plate (71), which may increase the amount of active species generated by a discharge. As a result, the air purifying properties of the discharge unit (20) may be improved.

The projection (49) of the insulating member (41) supports the longitudinal middle portion of the electrode support plate (71). Thus, the electrode support plate (71) may be stably supported by the insulating member (41), and the flatness of the electrode support plate (71) may be kept. As a result, the optimum distance between the discharge needles (73, 74) and the counter electrode (60) may be maintained.

The middle region (71a) of the electrode support plate (71) is supported by being sandwiched between the insulating member (41) and the stabilizer (80), which may simplify the support structure for the discharge needles (73, 74) and reduce the cost and time for forming the support structure. Further, this structure may prevent the discharge needles (73, 74) from bending and generate stable discharges.

The provision of the flanged portion (86) having the same electric potential as the discharge electrode (70) and opposed to the counter electrode (60) with respect to the discharge needles (73, 74) may stabilize the discharge directed to the counter electrode (60) from the discharge needles (73, 74). The conductive member (80) having the flanged portion (86) serves also as a member which sandwiches the electrode support plate (71) between itself and the insulating member (41). This structure may reduce the number of components.

The discharge needles (73, 74) and the counter electrode (60) may be positioned by fitting the projection (49) into the positioning hole (72) of the electrode support plate (71). In addition, the electrode support plate (71) may be supported on the insulating member (41) by a simple structure.

The warpage of the middle region (71a) may be more effectively prevented by fitting the elongated projection (49) into the elongated positioning hole (72) formed in the middle region (71a).

The insulating member (41) is provided with the flat mounting surface (50) formed on the support body (48) and surrounding the projection (49), and the electrode support plate (71) is placed on the mounting surface (50). The flatness of the electrode support plate (71) may thus be maintained, which may maintain the optimum distance between the discharge needles (73, 74) and the counter electrode (60).

The relative positions among the flanged portion (86), the discharge needles (73, 74), and the counter electrode (60) may be determined by inserting the projection (49) in the cylindrical wall portion (81) of the conductive member (80). As a result, more stable discharges may be generated from the discharge needles (73, 74) to the counter electrode (60). Further, the cylindrical wall portion (81) may be used as a spacer for keeping a distance between the flanged portion (86) and the discharge needles (73, 74).

The conductive member (80) and the insulating member (41) may be firmly fixed to each other by welding, using an ultrasonic wave, the fitted portion (49) fitted in the cylindrical wall portion (81). The discharge electrode (70) may thus be supported between the conductive member (80) and the insulating member (41) with reliability.

### -Variations of Embodiment-

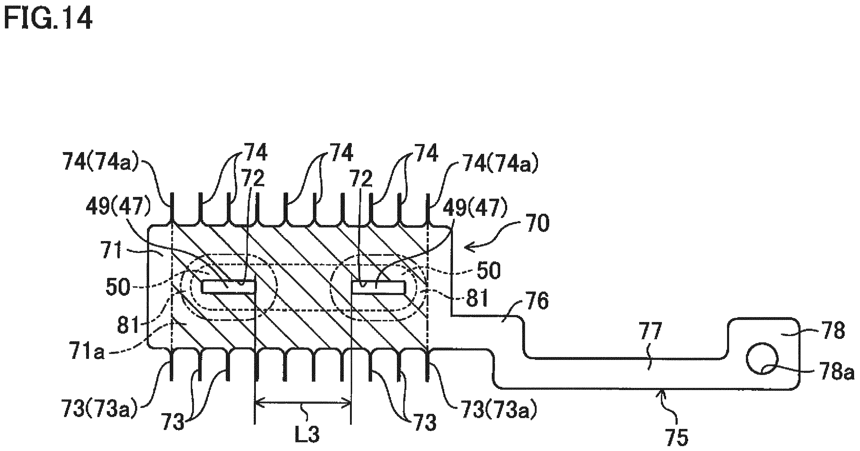

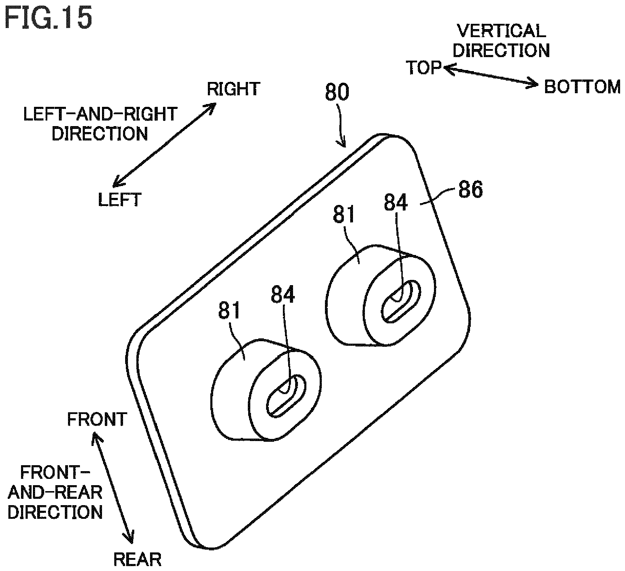

As illustrated in FIG. 14 and FIG. 15, the configurations of the insulating member (41), the discharge electrode (70), and the stabilizer (80) of the above-described embodiment may have the following variations.

A discharge process section (40) according to a variation is configured such that the support body (48) of the insulating member (41) has two projections (49). The projections (49) are arranged next to each other in the longitudinal direction of the support body (48). Thus, a flat mounting surface (50) is formed on the upper end surface of the support body (48) except the portions where the two projections (49) are provided.

The electrode support plate (71) of the discharge electrode (70) is provided with two positioning holes (72) at positions corresponding to the projections (49). The positioning holes (72) are arranged next to each other in the longitudinal direction of the electrode support plate (71). The projections (49) are fitted in the corresponding positioning holes (72) to place the electrode support plate (71) on the mounting surface (50) of the insulating member (41).

As illustrated in FIG. 15, the stabilizer (80) is provided with two cylindrical wall portions (81). The cylindrical wall portions (81) are located to correspond to the projections (49) and the positioning holes (72). The two projections (49) are inserted in the oblong openings (84) of the cylindrical wall portions (81), thereby having the stabilizer (80) supported on the insulating member (41). Each projection (49) in the corresponding cylindrical wall portion (81) is fixed to the stabilizer (80) or the discharge electrode (70) by ultrasonic wave welding in the same manner as in the above embodiment.

In this variation, too, the insulating member (41) supports the middle region (71a) of the electrode support plate (71), which may prevent warpage of the electrode support plate (71) due to temperature changes. It is recommended that the length L3 between the two positioning holes (72) as illustrated in FIG. 14 be 10 mm or less. If the length L3 is greater than 10 mm, the portion corresponding to this length may warp. The number of projections (49), the positioning holes (72), or the cylindrical wall portions (81) may be three or more.

The other advantages of the discharge unit (20) according to the variation are the same as, or similar to, those of the above-described embodiment.

### «Other Embodiments»

The above embodiment may also have the following structures.

The middle region (71a) of the electrode support plate (71) is provided with the opening (72) extending in the longitudinal direction of the electrode support plate (71). The opening (72) may have a circular or approximately square shape, and such openings (72) may be arranged in the longitudinal direction of the electrode support plate (71). In this case, a large number of openings (72) are formed in the middle region (71a), and a large number of projections (49) and cylindrical wall portions (81) may also be provided to correspond to these openings (72).

The air conditioner (10) of the above-described embodiment is installed on the back side of the ceiling (C). However, the discharge unit (20) of the present invention may be applied to an air conditioner of any other types, such as a wall-hung type, a ceiling-embedded type, and a ceiling-suspended type. The discharge unit (20) according to the present embodiment may be applied to an air cleaner.

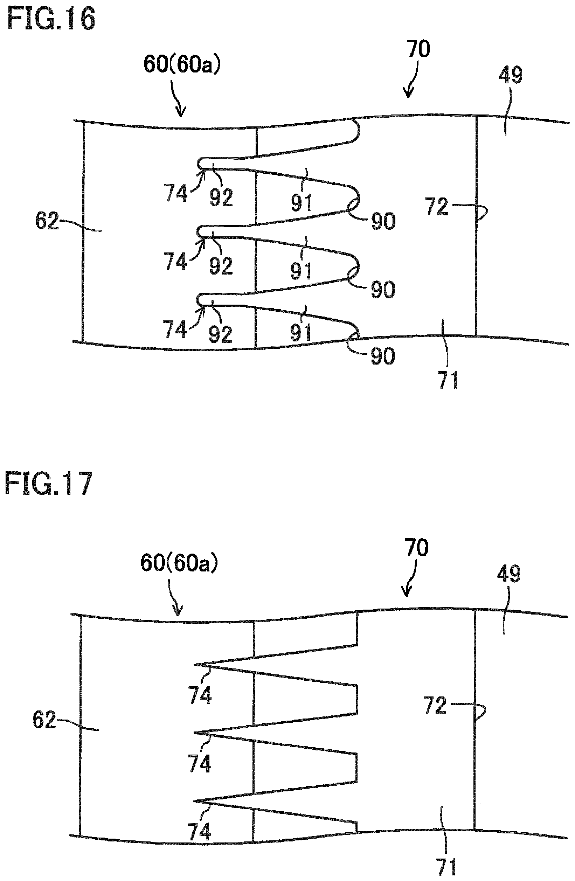

The discharge section of the above-described embodiment may be comprised of the discharge needles (73, 74) having a linear or rod-like elongated shape. However, the shape of the discharge section may not be limited to these shapes, and may have shapes illustrated in FIG. 16 to FIG. 18, which will be described below.

Each of a plurality of discharge sections (74) illustrated in FIG. 16 has a tapered plate portion (91) and a protruding plate portion (92). The discharge sections (74) are arranged along a side edge portion of the electrode support plate (71) to form a so-called comb-shaped electrode. The side edge portion of the electrode support plate (74) is provided with an arc-shaped recessed portion (90) between adjacent discharge sections (74). The width of the tapered plate portion (91) gradually decreases in a direction away from the side edge portion of the electrode support plate (71). The protruding plate portion (92) is continuous with the tip of the tapered plate portion (91) and protrudes further from the tip. The protruding plate portion (92) includes a body having a uniform width and a tip having an arc or semicircular shape. FIG. 16 depicts the discharge sections (74) on only one side, in the width direction, of the electrode support plate (71). However, the discharge sections (74) may be provided on either or both of the sides, in the width direction, of the electrode support plate (71).

Each of a plurality of discharge sections (74) illustrated in FIG. 17 has a sharp triangular shape (i.e., an isosceles triangular shape) which protrudes from a side edge portion of the electrode support plate (71). The discharge sections (74) are arranged along the side edge portion of the electrode support plate (71) to form a so-called saw-toothed electrode. FIG. 17 depicts the discharge sections (74) on only one side, in the width direction, of the electrode support plate (71). However, the discharge sections (74) may be provided on either or both of the sides, in the width direction, of the electrode support plate (71).

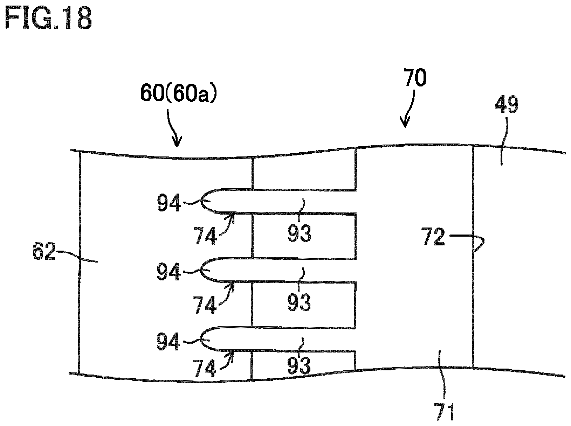

Each of a plurality of discharge sections (74) illustrated in FIG. 18 has a laterally-elongated rectangular plate portion (93) and an arc plate portion (94) on the tip of the rectangular plate portion (93). The plurality of discharge sections (74) are arranged along the side edge portion of the electrode support plate (71) to form a so-called saw-toothed electrode. The rectangular plate portion (93) has a uniform width from a proximal end to a distal end. The arc plate portion (94) is continuous with the tip of the rectangular plate portion (93) to have an arc, semicircular, elliptic arc, or other shape. FIG. 18 depicts the discharge sections (74) on only one side, in the width direction, of the electrode support plate (71). However, the discharge sections (74) may be provided on either or both of the sides, in the width direction, of the electrode support plate (71).

### INDUSTRIAL APPLICABILITY

As can be seen from the foregoing description, the present invention is useful as a discharge unit.

### DESCRIPTION OF REFERENCE CHARACTERS

- 20: Discharge Unit
- 30: Voltage Supply Section
- 41: Insulating Member
- 47: Support
- 48: Support Body
- 49: Projection (Fitted Portion)
- 50: Mounting Surface
- 60: Counter Electrode
- 70: Discharge Electrode
- 71: Electrode Support Plate
- 71a: Middle Region
- 72: Positioning Hole (Opening)
- 73: First Discharge Needle (Discharge Section)
- 74: Second Discharge Needle (Discharge Section)
- 80: Stabilizer (Conductive Member, Fixing Member)
- 81: Cylindrical Wall Portion
- 86: Flanged Portion

## Claims

1. A discharge unit comprising: a discharge electrode (70);
a counter electrode (60); and
a voltage supply section (30) configured to give a potential difference between the discharge electrode (70) and the counter electrode (60), wherein the discharge unit includes a resin-made insulating member (41) which supports the discharge electrode (70) and the counter electrode (60), the discharge electrode (70) is configured as a metal material and includes:
a plate-like electrode support plate (71); and
a plurality of discharge sections (73, 74) arranged along, and supported at, a side edge portion of the electrode support plate (71) so as to be opposed to the counter electrode (60), and
the insulating member (41) includes a support (47) which supports only a middle region (71a) of the electrode support plate (71) between two outermost discharge sections (73a, 74a) of the plurality of discharge sections (73,74);
a fixing member (80) which supports the discharge electrode (70) by sandwiching the middle region (71a) of the electrode support plate (71) between the fixing member (80) and the insulating member (41); and
the fixing member (80) is made of a conductive member (80) having a flanged portion (86), the flanged portion (86) being arranged opposite the counter electrode (60) with the discharge sections (73, 74) interposed there between and covering the entire discharge sections (73,74), being electrically connected to the discharge sections (73, 74) and having a shape along the discharge sections (73, 74).

2. The discharge unit of claim 1, wherein the electrode support plate (71) has an elongated shape extending in a direction of arrangement of the plurality of discharge sections (73,74) along the side edge portion of the electrode support plate (71).

3. The discharge unit of claim 1, wherein the middle region (71a) of the electrode support plate (71) is provided with an opening (72), and the insulating member (41) has a fitted portion (49) fitted in the opening (72).

4. The discharge unit of claim 3, wherein the opening (72) has an elongated shape extending in a direction of arrangement of the plurality of discharge sections (73, 74) along the side edge portion of the electrode support plate (71), and the fitted portion (49) has an elongated shape so as to be fitted in the opening (72).

5. The discharge unit of claim 3 or 4, wherein the support (47) includes a support body (48) and a projection (49) as the fitted portion which protrudes upward from an upper end of the support body (48), and a ring-shaped, planar mounting surface (50) surrounding the projection (49) is formed on the upper end of the support body (48), on which the electrode support plate (71) is mounted.

6. The discharge unit of any one of claims 3-5, wherein the conductive member (80) includes a cylindrical wall portion (81) in which the fitted portion (49) is fitted and which supports the flanged portion (86).

7. The discharge unit of claim 6, wherein the fitted portion (49) is fixed to the cylindrical wall portion (81) by ultrasonic wave welding.

## Patentansprüche

1. Entladungseinheit, umfassend: eine Entladungselektrode (70);
eine Gegenelektrode (60); und
einen Spannungsversorgungsabschnitt (30), der so konfiguriert ist, dass er eine Potentialdifferenz zwischen der Entladungselektrode (70) und der Gegenelektrode (60) erzeugt, wobei die Entladungseinheit ein aus Harz hergestelltes Isolierelement (41) enthält, das die Entladungselektrode (70) und die Gegenelektrode (60) trägt, wobei die Entladungselektrode (70) als ein metallisches Material konfiguriert ist und enthält:
eine plattenförmige Elektrodenträgerplatte (71); und
eine Vielzahl von Entladungsabschnitten (73, 74), die entlang eines Seitenkantenabschnitts der Elektrodenträgerplatte (71) angeordnet sind und dort gehalten werden, so dass sie der Gegenelektrode (60) gegenüberliegen, und
das Isolierelement (41) einen Träger (47) aufweist, der nur einen mittleren Bereich (71a) der Elektrodenträgerplatte (71) zwischen zwei äußersten Entladungsabschnitten (73a, 74a) der Vielzahl von Entladungsabschnitten (73, 74) trägt;
ein Befestigungselement (80), das die Entladungselektrode (70) durch sandwichartiges Anordnen des mittleren Bereichs (71a) der Elektrodenträgerplatte (71) zwischen dem Befestigungselement (80) und dem Isolierelement (41) trägt; und
das Befestigungselement (80) aus einem leitfähigen Element (80) mit einem mit einem Flansch versehenen Abschnitt (86) hergestellt ist, wobei der mit einem Flansch versehene Abschnitt (86) gegenüber der Gegenelektrode (60) mit den dazwischen angeordneten Entladungsabschnitten (73, 74) angeordnet ist und die gesamten Entladungsabschnitte (73, 74) abdeckt, mit den Entladungsabschnitten (73, 74) elektrisch verbunden ist und eine Form entlang der Entladungsabschnitte (73, 74) aufweist.

2. Entladungseinheit nach Anspruch 1, wobei die Elektrodenträgerplatte (71) eine längliche Form aufweist, die sich in einer Anordnungsrichtung der Vielzahl von Entladungsabschnitten (73, 74) entlang des Seitenkantenabschnitts der Elektrodenträgerplatte (71) erstreckt.

3. Entladungseinheit nach Anspruch 1, wobei der mittlere Bereich (71a) der Elektrodenträgerplatte (71) mit einer Öffnung (72) versehen ist und das Isolierelement (41) einen in die Öffnung (72) eingepassten Abschnitt (49) aufweist.

4. Entladungseinheit nach Anspruch 3, wobei die Öffnung (72) eine längliche Form aufweist, die sich in einer Anordnungsrichtung der Vielzahl von Entladungsabschnitten (73, 74) entlang des Seitenkantenabschnitts der Elektrodenträgerplatte (71) erstreckt, und der eingepasste Abschnitt (49) eine längliche Form aufweist, um in die Öffnung (72) eingepasst zu werden.

5. Entladungseinheit nach Anspruch 3 oder 4, wobei der Träger (47) einen Trägerkörper (48) und einen Vorsprung (49) als den eingepassten Abschnitt aufweist, der von einem oberen Ende des Trägerkörpers (48) nach oben vorsteht, und eine ringförmige, ebene Montagefläche (50), die den Vorsprung (49) umgibt, an dem oberen Ende des Trägerkörpers (48) ausgebildet ist, an dem die Elektrodenträgerplatte (71) montiert ist.

6. Entladungseinheit nach einem der Ansprüche 3 bis 5, wobei das leitfähige Element (80) einen zylindrischen Wandabschnitt (81) aufweist, in den der eingepasste Abschnitt (49) eingepasst ist und der den Flanschabschnitt (86) trägt.

7. Entladungseinheit nach Anspruch 6, wobei der eingepasste Abschnitt (49) an dem zylindrischen Wandabschnitt (81) durch Ultraschallwellenschweißen befestigt ist.

## Revendications

1. Un groupe de décharge composé des éléments suivants : une électrode de décharge (70) une contre-électrode (60) et
une section d'alimentation électrique (30) qui est configurée pour fournir une différence de potentiel entre l'électrode de décharge (70) et la contre-électrode (60), et ce groupe de décharge comporte un élément isolant en résine (41) qui soutient l'électrode de décharge (70) et la contre-électrode (60), et cette électrode de décharge (70) est configurée en tant que matériau métallique et comporte :
une plaque de soutien d'électrode du type plaquette (71) et
une pluralité de sections de décharge (73, 74) disposées le long d'une portion latérale de la plaque de soutien d'électrode (71) et soutenues par cette portion latérale, de manière à s'opposer à la contre-électrode (60) et
l'élément isolant (41) comporte un support (47) qui soutient uniquement une région médiane (71a) de la plaque de soutien d'électrode (71) entre deux sections externes de décharge (73a, 74a) de la pluralité de sections de décharge (73, 74)
un élément de fixation (80) qui soutient l'électrode de décharge (70) en encadrant la région médiane (71a) de la plaque de soutien d'électrode (71) entre l'élément de fixation (80) et l'élément isolant (41) et
l'élément de fixation (80) se compose d'un élément conducteur (80) qui a une partie à flasque (86), et cette partie à flasque (86) vient se positionner du côté opposé à la contre-électrode (60) et les sections de décharge (73, 74) viennent s'y intercaler et recouvrent la totalité des sections de décharge (73,74), et sont raccordées, sur le plan électrique, aux sections de décharge (73, 74) et suivent une pente le long des sections de décharge (73, 74).

2. Le groupe de décharge que décrit la revendication 1, si ce n'est que la plaque de soutien d'électrode (71) a un profil allongé qui se prolonge dans un sens qui correspond à un agencement composé d'une pluralité de sections de décharge (73, 74) le long de la partie latérale de cette plaque de soutien d'électrode (71).

3. Le groupe de décharge que décrit la revendication 1, si ce n'est que la région médiane (71a) de la plaque de soutien d'électrode (71) est équipée d'une ouverture (72) et que l'élément isolant (41) a une partie équipée (49) qui vient s'implanter dans cette ouverture (72).

4. Le groupe de décharge que décrit la revendication 3, si ce n'est que l'ouverture (72) a un profil allongé qui se prolonge dans un sens qui correspond à l'agencement composé de la pluralité de sections de décharge (73, 74) le long de la partie latérale de cette plaque de soutien d'électrode (71) et que la partie équipée (49) a un profil allongé qui lui permet de s'implanter dans cette ouverture (72).

5. Le groupe de décharge que décrit la revendication 3 ou 4, si ce n'est que le support (47) a un corps de support (48) et une partie saillante (49) qui se présente sous la forme de la partie implantée qui fait saillie vers le haut depuis une extrémité supérieure du corps du support (48), et une surface de montage planaire et de forme annulaire (50) qui entoure cette partie saillante (49) vient se former sur l'extrémité supérieure du corps du support (48), sur laquelle vient se monter la plaque support d'électrode (71).

6. Le groupe de décharge que décrit l'une ou l'autre des revendications 3 à 5, si ce n'est que l'élément conducteur (80) comporte une partie à cloison cylindrique (81) dans laquelle vient s'implanter la partie saillante (49) et qui soutient la partie à flasque (86).

7. Le groupe de décharge que décrit la revendication 6, si ce n'est que la partie saillante (49) vient se fixer sur la partie à cloison cylindrique (81) par soudage à ondes ultrasoniques.
